# EUROPÄISCHE PATENTANMELDUNG

(11) **EP 1 043 705 A1**
(43) Veröffentlichungstag der Anmeldung: **11.10.2000**
(21) Anmeldenummer: 99810296.6
(22) Anmeldetag: 09.04.1999
(51) Int. Cl.: G08B 5/22, A61B 5/0245

(54) **elektronische Vorrichtung zum Schutz von Patienten**

(71) Anmelder: Hagmann, Hansjörg, 5507 Mellingen (CH)
(72) Erfinder: Hagmann, Hansjörg, 5507 Mellingen (CH)

(57) **Zusammenfassung**

Die Erfindung lehrt eine Vorrichtung zur Überwachung von Patienten mittels am Korper angebrachten Sensoren, die bei übersehzeiten von zulässigen Grenzwerten eines Signals eine automatische Alarmmeldung zu einer Fernstation geben.

## Beschreibung

Die Erfindung betrifft eine Vorrichtung die den Zweck hat, Patienten in jeder Form zu überwachen.

Die bisher angewandte Technik zur Überwachung des Patienten war minimal. Der Patient konnte kaum, wenn er die Zeit oder die Kraft nicht mehr hatte Jemand rufen oder den Klingelknopf zu betätigen. Ausnahmen war in einer Intensivstation.

Der Erfindung liegt die Aufgabe zugrunde, auf elektronischem Weg, den Patienten immer automatisch zu überwachen.

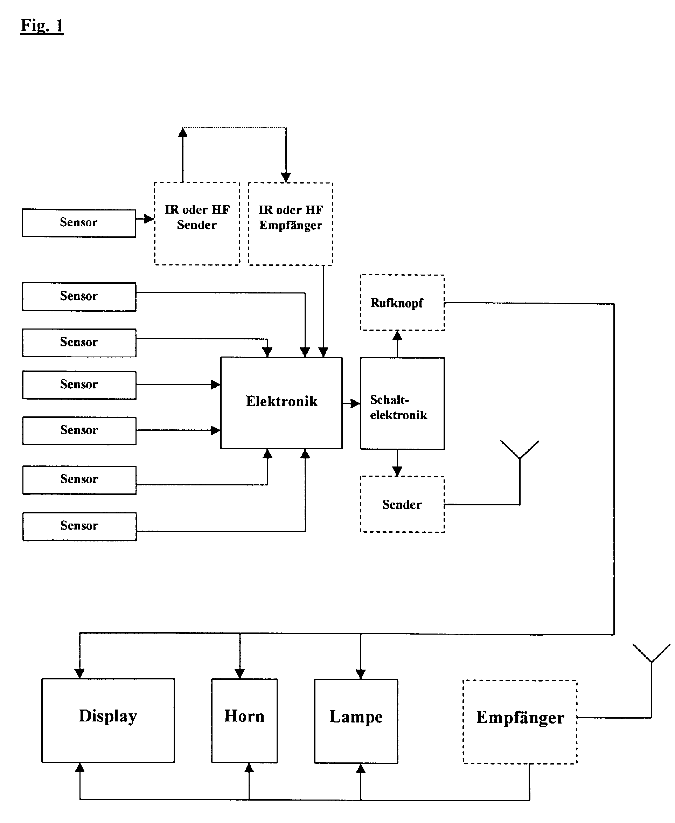

Erfindungsgemäss wird dies dadurch erreicht, dass je nach Patient mittels Sensor ein Alarm oder Klingelknopf ausgelöst wird, dies zeigt Figur 1.

Vorrichtung zum Schutz von Patienten, dadurch gekennzeichnet, dass Ein oder mehrere Sensoren den Patienten überwachen und bei einer Gefährdung Sofort automatisch der Klingelknopf oder Klingelknopfleitung benutzt wird.

## Patentansprüche

1. Vorrichtung nach Patentanspruch, dadurch gekennzeichnet, dass anstelle des Klingelknopfs oder Klingelknopfleitung auch eine andere Uebertragungsart wie Infrarot oder Funk möglich ist.

2. Vorrichtung nach Patentanspruch, dadurch gekennzeichnet, dass mittels Sensor der Puls, Blutdruck, Atem, Husten, Bettnässe, Fieber oder akustisch der Patient überwacht werden kann.

3. Vorrichtung nach Patentanspruch, dadurch gekennzeichnet, dass anstelle der aufleuchtenden Lampe oder akustischem Signal auch auf einem Panel optisch und akustisch der Patient ersichtlich ist.
